# EUROPEAN PATENT SPECIFICATION

(11) **EP 1 427 397 B1**
(45) Date of publication and mention of the grant of the patent: **13.02.2008**
(21) Application number: 02774546.2
(22) Date of filing: 02.09.2002
(51) Int. Cl.: A61K 9/48, A61K 47/10, A61K 47/14, A61K 47/02

(54) **OILY THIXOTROPIC FORMULATIONS**
ÖLIGE THIXOTROPE ZUSAMMENSETZUNGEN
FORMULATIONS THIXOTROPES HUILEUSES

(30) Priority: 10.09.2001 EP 01121545
(43) Date of publication of application: 16.06.2004
(73) Proprietor: F. HOFFMANN-LA ROCHE AG, 4002 Basel (CH)
(72) Inventor: KUENTZ, Martin, CH-4132 Muttenz (CH)
(74) Representative: Wächter, Dieter Ernst
(86) International application number: PCT/EP2002/009770
(87) International publication number: WO 2003/022254

(56) References cited:
- EP-A- 0 068 450
- EP-A- 1 035 115
- WO-A-00/01371
- WO-A-00/33866
- FR-A- 2 790 200
- GB-A- 1 590 864
- US-A- 4 450 877
- US-A- 5 665 384

## Description

The present invention relates to a novel thixotropic oily vehicle comprising a low amount of silicon dioxide and to a fill mass containing this vehicle. Furthermore, the present invention is directed to capsules, in particular hard gelatin capsules, filled with the above fill mass.

The term "capsule" encompasses hard and soft shell capsules which are preferably used to orally administer nutrients or pharmaceutically active ingredients to individuals. Such capsules are soluble under physiological conditions, digestible or permeable. The capsule shells are usually made of gelatin, starch, or other suitable physiologically acceptable macromolecular materials in form of gels. Examples thereof are soft gelatin capsules, hard gelatin capsules and Hydroxy Propyl Methyl Cellulose (HPMC) capsules.

The term "fill mass" defines one or more active compounds and/or nutrients and (possibly) suitable additives dissolved in a pharmaceutically acceptable vehicle.

The filling of liquid and semi-solid fill masses into capsules is widespread in the pharmaceutical industry. Especially the use of hard gelatin capsules becomes increasingly important because of certain characteristics making this dosage form even more preferred than that based on the soft gelatin technology. For example, hard gelatin shells are less sensitive towards heat and humidity and their permeability to oxygen is considerably lower than that of soft gelatin shells. Accordingly, hard gelatin capsules can be stored more easily and for a longer period of time without risking to damage the active compounds which they contain (see e.g. "Liquid Filled and Sealed Hard Gelatin Capsules", E.T. Cole, Bulletin Technique Gattefossé, 1999, p.70).

The use of hard gelatin capsules in the pharmaceutical industry is reviewed for instance in "Liquid Filling of Hard Gelatin Capsules: A New Technology for Alternative Formulations", W.J. Bowtle, Pharm. Technology Europe Oct. 1998, pp. 84-90.

The possibility of using capsules as unit dose for administering nutrients or pharmaceutical active ingredients depends on the flow behavior of the fill mass which has to be encapsulated. Ideally, the fill mass should be liquid during the filling process while it should solidify or become a gel once encapsulated.

It is advantageous that solidification or gelling of the fill mass occurs since, in this way, a final sealing step of the capsule shell can be avoided. For suspensions, a gelification with a relatively high yield point (i.e. the critical stress to induce plastic deformation of the material, measured in Pa) is even vital to prevent re-liquefaction by accidental shaking of the capsules during e.g. transportation. Accidental re-liquefaction of the fill mass after encapsulation would in fact cause settling and caking of the suspended e.g. drug particles, thus decreasing dissolution and possibly also the bioavailability.

Such an ideal flow performance can be obtained by melting a waxy formulation during filling or by providing a so-called thixotropic system. The thixotropy is the property of certain solids or gels, which liquefy when subjected to shear forces and then solidify again when left standing. Thixotropic systems do not involve heat treatments and are therefore especially suitable for thermolabile active pharmaceutical substances. The absence of a heating phase is also favorable for suspensions where increased drug solubility may result to a precipitation upon cooling.

The particular characteristics of the thixotropic systems in the context of pharmaceutical fill masses are e.g. highlighted in "The filling of molten and thixotropic formulations into hard gelatin capsules", S.E. Walker, J.A. Ganley, K. Bedford and T. Eaves, J. Pharm.Pharmacol. 32, 1980, pp. 389-393.

On the other hand, many substances obtained from modern drug discovery are problematic in view of a sufficient bioavailability and often exhibit a very low aqueous solubility so that they have to be formulated in oily (apolar) vehicles.

Unfortunately, there are only few excipients originating thixotropy in oily systems whereby the most significant is silicon dioxide. These colloidal silica display thixotropy and a convenient yield point (> 2-4 Pa) at concentrations of about 4 - 10% (w/w) depending on the polarity of the oil.

The viscosity under shear of the thixotropic vehicle, which is measured at a defined shear rate, must be enough low (< 300 mPa s) to also enable the filling of high concentrated suspensions where the viscosity is often the limiting factor of the technical feasibility. However, suspensions with a high amount of solid phase have to be processed to guarantee the possibility of widely varying the drug load range of the final dosage form.

It is furthermore necessary to keep the concentration of silicon dioxide in the fill mass as low as possible since this colloidal powder is exceptionally bulky (density is = 0.03 g/cm³) and potentially harmful upon inhalation. The use of this material on an industrial scale may raise several practical problems and may endanger the health of the technicians who work with it.

The problem at the root of the present invention is therefore to provide a thixotropic oily vehicle containing as little silicon dioxide as possible but showing both a high yield point (> 4 Pa) and a low viscosity under shear (< 300 mPa s).

This problem is solved, according to the present invention, by providing a thixotropic oily vehicle comprising from 0.2% to 5% (w/w) of a colloidal silica and from 0.2% to 5% (w/w) of a hydrophilic polymer.

The oily vehicle according to the present invention contains a reduced amount of silicon dioxide, while showing a relatively elevated yield point, a high thixotropy and a low viscosity under shear. The decrease of silicon dioxide quantities is meaningful regarding the reduction of the bulk volume when it is processed on a production scale.

The interaction between the hydrophylic polymer and the colloidal silicon dioxide in the above concentration ranges enables to keep the amount of the latter component at a low concentration, by nevertheless conferring on the solution enough thixotropy and a low viscosity under shear.

The positive effects of this interaction are quite surprising and unexpected. In fact, although it is known that additives may improve the thickening performance of the colloidal silica dioxide (see e.g. Degussa's Technical Bulletin No. 23: "Aerosil® as a Thickening Agent for Liquid Systems", 1989, pp. 22-24) it is to be expected that the addition of a hydrophilic polymer leads to a phase separation in the apolar oily environment, rather than to a homogenous colloidal system. In the claimed concentrations, however, the interaction of the silica surface with the hydrophilic polymer builds a coherent structure meeting the desired flow performance for liquid-fill systems.

When left standing, the composition of the present invention has preferably the visual aspect of a transparent oily gel.

According to a preferred embodiment of this invention, the colloidal silica is chosen from the group consisting of Aerosil^{®} 200, Aerosil^{®} 300 and Aerosil^{®} R812 (Degussa AG, Frankfurt) whereby the most preferred colloidal silica is Aerosil^{®} 200. The colloidal silica is preferably used in a concentration varying from 0.5% to 3% (w/w) and, still more preferably, in a concentration varying from 1% to 2% (w/w).

The hydrophilic polymer used according to the present invention can be chosen from the group of polyethers and polyalcohols. Examples thereof are the polyethylene glycols, the polypropylen polyethylene glycols and the polyvinylalcohols. Preferred are the polyethylene glycols having a molecular weight equal to or smaller than 400 g/mol. Examples thereof are polyethylene glycol 200, polyethylene glycol 300 and polyethylene glycol 400. Most preferred is the polyethylene glycol 300.

The hydrophilic polymer is advantageously present in a concentration varying from 0.5% to 4% (w/w) and, still more advantageously, in a concentration varying from 1% to 3% (w/w).

As stated above, the oily vehicle of the present invention is suitable for the preparation of liquid-filled capsules which are intended for oral drug delivery. It is particularly suitable for active compounds whose oral bioavailability and/or chemical stability can be improved by a lipidic formulation rather than by a conventional dosage form. The special pharmacokinetic profile of a certain active compound can be a further reason to use a lipidic vehicle as dispering medium. Examples of such active compounds can be found among esters, lactones, retinoids, steroids, dihydropyridins and 4-phenylpyridin derivatives. Particularly, the present composition is used for active compounds selected among the group of the 4-phenylpyridine derivatives such as:
2-(3,5-bis-trifluoromethyl-phenyl)-N-methyl-N-(6-morpholin-4-yl-4-o-tolyl-pyridin-3-yl)-isobutyramide;
2-(3,5-bis-trifluoromethyl-phenyl)-N-methyl-N-[6-(4-methyl-piperazin-1-yl)-4-o-tolyl-pyridin-3-yl]-isobutyramide; and
2-(3,5-bis-trifluoromethyl-phenyl)-N-[4-(2-chloro-phenyl)-pyridin-3-yl]-N-methyl-isobutyramide.

The above three compounds, whose synthesis may be found in EP-A-1035115, are characterized by valuable therapeutic properties. They are highly selective antagonists of the Neurokinin 1 (NK-1, substance P) receptor. Substance P is a naturally occurring undecapeptide belonging to the tachykinin family of peptides, the latter being so-named because of their prompt contractile action on extravascular smooth muscle tissue.

The oily component of the vehicle according to the present invention consists in an edible oil which can be chosen from the natural and semi-synthetic vegetable mono-, di- or triglycerides. Preferred are pharmaceutical grade triglycerides oils such as corn oil, peanut oil, olive oil, castor oil, or middle chain triglyceride oil (Miglyol) or mixtures thereof. Most preferred is the middle chain triglyceride oil (Miglyol).

The present invention is also directed to a process for preparing a thixotropic oily vehicle as described above, which process comprises mixing, in an edible oil as defined above, from 0.2% to 5% (w/w) of a colloidal silica with from 0.2% to 5% (w/w) of a hydrophilic polymer.

A further aspect of the present invention consists in providing a fill mass comprising a thixotropic oily vehicle as described above and one or more pharmaceutically active ingredients.

A still further aspect of the present invention is directed to pharmaceutical unit dose wherein a fill mass as described above is encapsulated in an edible capsule. According to a preferred embodiment, the capsule is made of gelatin and, still more preferably, of hard gelatin.

The present invention is further described by the following non-limiting examples. Table 1 shows the viscosity under shear and the yield point of the exemplified oily vehicles, as well as of comparative oily vehicles which do not include a hydrophilic polymer.

The rheological characterization was performed using a controlled stress instrument Carri-Med CSL 500 equipped with a cone and plate system (6 cm diameter and 2° angle). The viscosity was determined at a shear rate of 100 s⁻¹ and a temperature of 25 °C on the "down-curve" of the hysteresis flow curve. On the other hand, the "up-curve" was used to extrapolate the yield point according to the *Casson* model ("Das Rheologie Handbuch fur Anwender von Rotations- und Oszillations-Rheometern", T. Mezger, Vincentz, 2000, p.54).

### PREPARATIONS OF THE COMPOSITION

### Example 1

2.0 g Aerosil^{®} 200 were exactly weighted and dispersed with a mixer (Type Bamix® (Switzerland), level 2 during 30 seconds) in 96.0 g of Miglyol 812 (middle chain triglyceride). 2.0 g of fluid polyethylene glycol 400 were added to and mixed with the above suspension (Bamix, level 2 during 45 seconds). The so obtained thixotropic vehicle was finally put under vacuum to remove the incorporated air.

### Example 2

The procedure of Example 1 was repeated with the following composition:

| | |
|---|---|
| 1.5 g | Aerosil^{®} 200 |
| 2.0 g | Polyethylene glycol 300 |
| 96.5 g | Miglyol 812 (middle chain triglyceride) |

### Example 3

The procedure of Example 1 was repeated with the following composition:

| | |
|---|---|
| 2.0 g | Aerosil^{®} 200 |
| 2.5 g | Polyethylene glycol 300 |
| 95.5 g | Miglyol 812 (middle chain triglyceride) |

### Example 4

The procedure of Example 1 was repeated with the following composition:

| | |
|---|---|
| 1.5 g | Aerosil^{®} 200 |
| 2.0 g | Polyethylene glycol 300 |
| 96.5 g | Peanut oil |

### Example 5

The procedure of Example 1 was repeated with the following composition:

| | |
|---|---|
| 5.0 g | 2-(3,5-bis-trifluoromethyl-phenyl)-N-methyl-N-(6-morpholin-4-yl-4-o-tolyl-pyridin-3-yl)-isobutyramide. |
| 1.5 g | Aerosil^{®} 200 |
| 1.0 g | Polyethylene glycol 300 |
| 92.5 g | Miglyol 812 (middle chain triglyceride) |

### Example 6

The procedure of Example 1 was repeated with the following composition:

| | |
|---|---|
| 5.0 g | 2-(3,5-bis-trifluoromethyl-phenyl)-N-methyl-N-(6-morpholin-4-yl-4-o-tolyl-pyridin-3-yl)-isobutyramide. |
| 1.5 g | Aerosil^{®} 200 |
| 2.0 g | Polyethylene glycol 300 |
| 91.5 g | Miglyol 812 (middle chain triglyceride) |

### Example 7

The procedure of Example 1 was repeated with the following composition:

| | |
|---|---|
| 5.0 g | 2-(3,5-bis-trifluoromethyl-phenyl)-N-methyl-N-(6-morpholin-4-yl-4-o-tolyl-pyridin-3-yl)-isobutyramide. |
| 1.5 g | Aerosil^{®} 200 |
| 3.0 g | Polyethylene glycol 300 |
| 90.5 g | Miglyol 812 (middle chain triglyceride) |

### Example C1 (Comparative)

The procedure of Example 1 was repeated with the following composition:

| | |
|---|---|
| 2.0 g | Aerosil^{®} 200 |
| 98.0 g | Miglyol 812 (middle chain triglyceride) |

### Example C2 (Comparative)

The procedure of Example 1 was repeated with the following composition:

| | |
|---|---|
| 5.0 g | Aerosil^{®} 200 |
| 95.0 g | Miglyol 812 (middle chain triglyceride) |

### Example C3 (Comparative)

The procedure of Example 1 was repeated with the following composition:

| | |
|---|---|
| 6.0 g | Aerosil^{®} 200 |
| 94.0 g | Miglyol 812 (middle chain triglyceride) |

### Example C4 (Comparative)

| | |
|---|---|
| 5.0 g | 2-(3,5-bis-trifluoromethyl-phenyl)-N-methyl-N-(6-morpholin-4-yl-4-o-tolyl-pyridin-3-yl)-isobutyramide. |
| 1.5 g | Aerosil^{®} 200 |
| 93.5 g | Miglyol 812 (middle chain triglyceride) |

**Table 1**

| Rheological Characterization | | | | |
|---|---|---|---|---|
| Ex. | Amount of Aerosil^{®} 200 (% w/w) | Amount of polyethylene glycol (% w/w) | Viscosity (100s⁻¹ /25 °C) (mPa s) | Yield point (Pa) |
| 1 | 2.0 | 2.0 | 55 | 8.30 |
| 2 | 1.5 | 2.0 | 137 | 7.13 |
| 3 | 2.0 | 2.5 | 207 | 17.08 |
| 4 | 1.5 | 2.0 | 249 | 7.23 |
| 5 | 1.5 | 1.0 | 205 | 5.01 |
| 6 | 1.5 | 2.0 | 149 | 4.67 |
| 7 | 1.5 | 3.0 | 135 | 4.68 |
| C1 | 2.0 | - | 56 | 0.14 |
| C2 | 5.0 | - | 201 | 4.00 |
| C3 | 6.0 | - | 349 | 9.07 |
| C4 | 1.5 | - | 59 | 0.11 |

As it can be seen from Table 1, the addition of a hydrophilic polymer (polyethylene glycol) enables to decrease the amount of colloidal silica necessary to confer to the oily vehicle a sufficiently high yield point (at least 4 Pa), by keeping the viscosity under shear below 300 mPa s. Without adding the hydrophilic polymer, yield points above 4 can be obtained only at Aerosil^{®} concentrations of 5% (w/w) or more.

If Example 2 and Example C2 are compared, it can be seen that the addition of 2% (w/w) of polyethylene glycol enables to decrease the amount of Aerosil^{®} by a factor 3.33 (w/w) and still to have an almost doubled yield point (7.13 vs. 4 Pa) and a lower viscosity under shear (137 vs. 201 mPa s).

Other comparisons from Table 1 between the vehicles according to the present invention and the conventional ones (e.g. Ex 1 with Ex C1) demonstrate that, at a Aerosil^{®} concentration of 2%, the addition of a hydrophilic polymer enables to strongly increase the yield point (0.14 vs. 8.30 Pa).

## Claims

1. Thixotropic oily vehicle comprising from 0.2% to 5% (w/w) of a colloidal silica and from 0.2% to 5% (w/w) of a hydrophilic polymer in an edible oil.

2. The vehicle according to claim 1, wherein the colloidal silica is present in a concentration varying from 0.5% to 3% (w/w).

3. The vehicle according to claim 2, wherein the colloidal silica is present in a concentration varying from 1% to 2% (w/w).

4. The vehicle according to any one of the preceding claims, wherein the colloidal silica is chosen from the group consisting of Aerosil^{®} 200, Aerosil^{®} 300 or Aerosil^{®} R812.

5. The vehicle according to claim 4, wherein the colloidal silica is Aerosil^{®} 200.

6. The vehicle according to any one of the preceding claims, wherein the hydrophilic polymer is present in a concentration varying from 0.5% to 4% (w/w).

7. The vehicle according to claim 6, wherein the hydrophilic polymer is present in a concentration varying from 1% to 3% (w/w).

8. The vehicle according to any one of the preceding claims, wherein the hydrophilic polymer is chosen from the group consisting of polyethers and polyalcohols.

9. The vehicle according to claim 8, wherein the hydrophilic polymer is a polyethylene glycol having a molecular weight equal or smaller than 400 g/mol.

10. The vehicle according to claim 9, wherein the hydrophilic polymer is polyethylene glycol 300.

11. The vehicle according to any one of the preceding claims, wherein the edible oil is chosen from the group consisting of natural and semi-synthetic vegetable mono, di- and triglyceride.

12. The vehicle of claim 11, wherein the edible oil is a triglycerid oil.

13. The vehicle of claim 12, wherein the triglycerid oil is chosen from the group consisting of corn oil, peanut oil, olive oil, castor oil, and middle chain triglyceride oil.

14. The vehicle of claim 13, wherein the triglycerid oil is the middle chain triglyceride oil.

15. Process for preparing a thixotropic oily vehicle according to any one of claims 1 to 14 comprising mixing, in an edible oil, from 0.2% to 5% (w/w) of a colloidal silica with from 0.2% to 5% (w/w) of a hydrophilic polymer.

16. Fill mass comprising a thixotropic oily vehicle according to any one of claims 1 to 14.

17. Pharmaceutical unit dose comprising a fill mass according to claim 16, encapsulated in an edible capsule.

18. The pharmaceutical unit dose of claim 17, wherein the capsule is made of gelatin.

19. The pharmaceutical unit dose of claim 18, wherein the capsule is made of hard gelatin.

## Patentansprüche

1. Thixotropes öliges Vehikel, umfassend 0,2 bis 5 Gew.-% eines kolloiden Siliciumdioxids und 0,2 bis 5 Gew.-% eines hydrophilen Polymers in einem Speiseöl.

2. Vehikel nach Anspruch 1, wobei das kolloide Siliciumdioxid in einer Konzentration, die von 0,5 bis 3 Gew.-% variiert, vorliegt.

3. Vehikel nach Anspruch 2, wobei das kolloide Siliciumdioxid in einer Konzentration, die von 1 bis 2 Gew.-% variiert, vorliegt

4. Vehikel nach einem der vorhergehenden Ansprüche, wobei das kolloide Siliciumdioxid aus der Gruppe ausgewählt ist, bestehend aus Aerosil^{®} 200, Aerosil^{®} 300 oder Aerosil^{®} R812.

5. Vehikel nach Anspruch 4, wobei das kolloide Siliciumdioxid Aerosil^{®} 200 ist.

6. Vehikel nach einem der vorhergehenden Ansprüche, wobei das hydrophile Polymer in einer Konzentration, die von 0,5 bis 4 Gew.-% variiert, vorliegt.

7. Vehikel nach Anspruch 6, wobei das hydrophile Polymer in einer Konzentration, die von 1 bis 3 Gew.-% variiert, vorliegt.

8. Vehikel nach einem der vorhergehenden Ansprüche, wobei das hydrophile Polymer aus der Gruppe ausgewählt ist, bestehend aus Polyethern und Polyalkoholen.

9. Vehikel nach Anspruch 8, wobei das hydrophile Polymer ein Polyethylenglycol mit einem Molekulargewicht gleich oder kleiner als 400 g/mol ist.

10. Vehikel nach Anspruch 9, wobei das hydrophile Polymer Polyethylenglycol 300 ist.

11. Vehikel nach einem der vorhergehenden Ansprüche, wobei das. Speiseöl aus der Gruppe ausgewählt ist, bestehend aus natürlichem und halbsynthetischem, pflanzlichem Mono-, Di- und Triglycerid.

12. Vehikel nach Anspruch 11, wobei das Speiseöl ein Triglyceridöl ist.

13. Vehikel nach Anspruch 12, wobei das Triglyceridöl aus der Gruppe ausgewählt ist, bestehend aus Maisöl, Erdnußöl, Olivenöl, Rizinusöl und Triglyceridöl mit mittlerer Kettenlänge.

14. Vehikel nach Anspruch 13, wobei das Triglyceridöl das Triglyceridöl mit mittlerer Kettenlänge ist.

15. Verfahren zur Herstellung eines thixotropen öligen Vehikels nach einem der Ansprüche 1 bis 14, umfassend das Mischen von 0,2 bis 5 Gew.-% eines kolloiden Siliciumdioxids mit 0,2 bis 5 Gew.-% eines hydrophilen Polymers in einem Speiseöl.

16. Füllmasse, umfassend ein thixotropes öliges Vehikel nach einem der Ansprüche 1 bis 14.

17. Pharmazeutische Einheitsdosis, umfassend eine Füllmasse nach Anspruch 16, die in eine eßbare Kapsel eingekapselt ist.

18. Pharmazeutische Einheitsdosis nach Anspruch 17, wobei die Kapsel aus Gelatine ist.

19. Pharmazeutische Einheitsdosis nach Anspruch 18, wobei die Kapsel aus harter Gelatine ist.

## Revendications

1. Véhicule huileux thixotrope comprenant de 0,2 % à 5 % en masse d'une silice colloïdale et de 0,2 % à 5 % en masse d'un polymère hydrophile dans une huile comestible.

2. Véhicule selon la revendication 1, dans lequel la silice colloïdale est présente à une concentration variant de 0,5 % à 3 % en masse.

3. Véhicule selon la revendication 2, dans lequel la silice colloïdale est présente à une concentration variant de 1 % à 2 % en masse.

4. Véhicule selon l'une quelconque des revendications précédentes, dans lequel la silice colloïdale est choisie dans le groupe constitué de l'Aerosil^{®} 200, l'Aerosil^{®} 300 ou l'Aerosil^{®} R812.

5. Véhicule selon la revendication 4, dans lequel la silice colloïdale est l'Aerosil^{®} 200.

6. Véhicule selon l'une quelconque des revendications précédentes, dans lequel le polymère hydrophile est présent à une concentration variant de 0,5 % à 4 % en masse.

7. Véhicule selon la revendication 6, dans lequel le polymère hydrophile est présent à une concentration variant de 1 % à 3 % en masse.

8. Véhicule selon l'une quelconque des revendications précédentes, dans lequel le polymère hydrophile est choisi dans le groupe constitué des polyéthers et des polyalcools.

9. Véhicule selon la revendication 8, dans lequel le polymère hydrophile est le polyéthylèneglycol ayant une masse moléculaire égale ou inférieure à 400 g/mol.

10. Véhicule selon la revendication 9, dans lequel le polymère hydrophile est le polyéthylèneglycol 300.

11. Véhicule selon l'une quelconque des revendications précédentes, dans lequel l'huile comestible est choisie dans le groupe constitué des mono-, di- et triglycérides végétaux naturels et semi-synthétiques.

12. Véhicule selon la revendication 11, dans lequel l'huile comestible est une huile triglycéride.

13. Véhicule selon la revendication 12, dans lequel l'huile triglycéride est choisie dans le groupe constitué de l'huile de maïs, l'huile d'arachide, l'huile d'olive, l'huile de ricin et une huile triglycéride à chaîne moyenne.

14. Véhicule selon la revendication 13, dans lequel l'huile triglycéride est l'huile triglycéride à chaîne moyenne.

15. Procédé de préparation d'un véhicule huileux thixotrope selon l'une quelconque des revendications 1 à 14, comprenant le mélange, dans une huile comestible, de 0,2 % à 5 % en masse d'une silice colloïdale avec de 0,2 % à 5 % en masse d'un polymère hydrophile.

16. Masse de remplissage comprenant un véhicule huileux thixotrope selon l'une quelconque des revendications 1 à 14.

17. Dose unitaire pharmaceutique comprenant une masse de remplissage selon la revendication 16, encapsulée dans une capsule comestible.

18. Dose unitaire pharmaceutique selon la revendication 17, dans laquelle la capsule est en gélatine.

19. Dose unitaire pharmaceutique selon la revendication 18, dans laquelle la capsule est en gélatine dure.
